# EUROPEAN PATENT SPECIFICATION

(11) **EP 2 528 591 B1**
(45) Date of publication and mention of the grant of the patent: **03.01.2018**
(21) Application number: 11708944.1
(22) Date of filing: 28.01.2011
(51) Int. Cl.: A61K 9/16, A61K 9/20, A61P 11/00, A61P 13/00, A61K 31/545, A61P 31/00

(54) **EFFERVESCENT FORMULATIONS COMPRISING CEFACLOR**
BRAUSEFORMULIERUNG ENTHALTEND CEFACLOR
FORMULATIONS EFFERVESCENTES COMPRENANT DU CÉFACLOR

(30) Priority: 29.01.2010 TR 201000688
(43) Date of publication of application: 05.12.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000028
(87) International publication number: WO 2011/093824

(56) References cited:
- EP-A1- 0 862 915
- WO-A1-99/49868
- WO-A1-2007/086012
- CN-C- 100 417 383

## Description

Present invention relates to pharmaceutical formulations in effervescent form comprising cefaclor.

### Prior Art

Cefaclor is a semi-synthetic, wide spectrum, second generation cephalosporin antibiotic which is shown with Formula (I) and which is disclosed in US3925372 (A) for the first time by Eli Lilly. 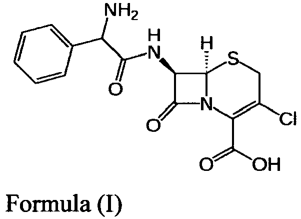 Cefaclor, which is physically in white or slightly yellow and in the powder form, slightly dissolve in water but does not dissolve in methanol and methylene chloride. The product sold in the market under the tradename CECLOR® is present in capsule and suspension forms.
Cefaclor is used for treatment of infections caused by gram positive microorganisms; staphylococcus aureus, Staphylococcus epidermidis, Staphylococcus saprophyticus, Staphylococcus pneumonia, Streptococcus pyogenes (group A streptococci), gram negative microorganisms; Haemophilus parainfluenzea, Haemophilus influenzae, Moraxella (Branhamella) catarrhalis, Escherichia coli, Klebsiella pneumonia, respiratory tract infections caused by Proteus mirabilisin such as (pneumonia, bronchitis, acute inflammations of chronic bronchitis, pharangitis, tonsilitis), middle-ear infections, sinusitis, skin and soft tissue infections and urinary tract infections (cystitis and pyelonephritis).
Formulations comprising cefaclor are commonly present in the forms of tablet, oral suspension and capsule. WO 99/49868 discloses a sustained-release composition comprising cefaclor. These dosage forms, where large amounts of antibiotic are formulated with the excipients, become bigger in size and this affects the usage negatively. Alternatively, the use of reliable and user-friendly effervescent forms is suggested.

In the patent application numbered in WO2006078241, the manufacture of a medicament comprising a cephalosporin antibiotic like cephalothin in the effervescent form is disclosed. In this patent application, compared to the total weight of the composition, maximum 1.5%, preferably 0.5-1% of lubricant is used and additionally maximum 60%, preferably 10-40% of binder is used.

However, when effervescent formulations comprising cefaclor as active agent and said amounts of lubricant and binder are prepared, the smooth flow of the mixture can not be obtained since the free flow of granules is not provided and the possible friction between the interface of the tablets is not decreased. Therefore, in cases where the formulations are compressed into tablets, this problem causes a change in tablet weight and increase the relative standard deviation. The weight uniformity can not be provided due to the observed deviations in tablet weight and this causes some problems for the producer in the production and quality control stages.

As is seen, it is necessary to provide weight uniformity for an effective treatment and any problem related to the weight uniformity observed in production and quality control stages should be prevented. For this purpose, it is necessary to develop new approaches for obtaining the drug in effervescent form in which inappropriate changes in unit dosage weight will not be observed.

As a result of the studies toward this requirement, the inventors have found that the developed formulations for the preparation of effervescent forms comprising cefaclor have an effect on the relative standard deviation of the drug dosage weight and the problems in the prior art can be solved by these formulations.

### Description of the Invention

The present invention is related to effervescent formulations comprising cefaclor and process for the preparation of these formulations. Surprisingly, when the formulations, in which the amount of lubricant used in the granulation solution is in the range of 2-10%, preferably 2-8%, more preferably 2-5% and additionally the amount of binder is in the range of 0.3-8%, preferably 0.35-7%, more preferably 0.4-4%, are prepared in solid dosage form, preferably tablet form, it is seen that the relative standard deviation decreases and the weight uniformity can be provided by preventing the changes in the weight of effervescent dosage form.

The inventors have studied the relative standard deviation of dosage weight by changing the ratios of lubricant and binder in order to find the effect of the lubricant and binder ratios on the changes of weight and thus the weight uniformity. Accordingly, the ratios of lubricant and binder compared to the total weight of unit dosage and the values of relative standard deviation (RSD%) are given in Table 1. As it is seen in Table 1, the values of RSD% are obtained as under 2% when the amount of binder is in the range of 0.4-4 % and the amount of lubricant is in the range of 2-5 %. Therefore, when the lubricant and binder are used in said amounts in the granulation solution, they have found that the value of relative standard deviation is low and the weight unformity is provided.

**Table 1: The lubricant and binder ratios compared to the total weight of the unit dosage and accordingly the values of relative standard deviation.**

| Binder % amount | Lubricant % amount | % RSD |
|---|---|---|
| 0.1 | 0.5 | 7.5 |
| 0.2 | 1 | 6.3 |
| 0.3 | 1.5 | 3.7 |
| 0.4 | 2 | 2.5 |
| 0.5 | 2.5 | 1.3 |
| 0.6 | 2.75 | 1.2 |
| 0.8 | 3.25 | 1.35 |
| 1 | 3.75 | 1.4 |
| 1.5 | 4 | 1.6 |
| 2 | 4.25 | 1.45 |
| 3 | 4.75 | 1.9 |
| 4.5 | 5.5 | 3.2 |
| 5.5 | 7.5 | 4.1 |

Accordingly, the first aspect of the present invention is that the granulation solution prepared in the effervescent formulations comprising cefaclor comprises 2-10%, preferably 2-8%, more preferably 2-5% of lubricant and 0.3-8%, preferably 0.35-7%, more preferably 0.4-4% of binder.

Binder used in the effervescent formulation in accordance with the present invention can be selected from, but not limited with, a group comprising; alginic acid, chitosan, carbomer, carboxymethyl cellulose sodium, dextrin, hydroxyethyl cellulose, hydroxymethyl cellulose, hydroxypropyl cellulose, ethyl cellulose, gelatine, hypromellose, magnesium aluminium silicate, maltodextrin, polyethylene oxide and povidone or a combination thereof. Preferably povidone is used.

Lubricant used in the effervescent formulation in accordance with the present invention can be selected from a group comprising PEG 6000, sodium benzoate, calcium stearate, glyceryl monostearate, magnesium stearate, polyvinyl alcohol, potassium benzoate, stearic acid and talc. Preferably, PEG 6000 is used.

In another aspect, the present invention is related to the effervescent formulations in which granulation solution that is used for the preparation of said formulations comprises 2-10%, preferably 2-8%, more preferably 2-5% of PEG 6000 and 0.3-8%, preferably 0.35-7%, more preferably 0.4-4% of povidone.

In the effervescent formulation in accordance with the present invention, cefaclor and/or its pharmaceutically acceptable salts, hydrates, solvates and/or a combination thereof can be used.

Cefaclor that is used in the present invention can be present in one of the forms of monohydrate, dihydrate, trihydrate and/or anhydrous. Preferably, it is used in monohydrate form.

Another aspect of the present invention is that the effervescent formulation comprises cefaclor in an amount in the range of 1-55 %, preferably 5-50 % and more preferably 10-40 % compared to the total dosage weight.

Another aspect of the present invention is phamaceutical composition comprising cefaclor, at least one binder, at least one lubricant and in addition to these at least one pharmaceutically acceptable excipient.

Pharmaceutical composition formulated in the effervescent form in accordance with the present invention comprises cefaclor as active agent, at least one binder, preferably povidone, at least one lubricant, preferably PEG 6000 and additionally one or more of pharmaceutically acceptable excipients selected from a group comprising effervescent couple including effervescent acid and base, sweetener and/or taste regulator, flavoring agent, glidant, diluents, disintegrant, coloring agent, surfactant, anti-foaming agent, humectants, acidic agent and basic agent.

Effervescent acid that is used in the effervescent formulation according to the present invention is selected from a group comprising citric acid, tartaric acid, malic acid, fumaric acid, ascorbic acid, acetic acid, adipic acid, succinic acid, acetylsalicylic acid and/or sodium citrate, sodium acetate, sodium dihydrogen phosphate, sodium acid pyrophosphate and sodium acid sulphite. Preferably, citric acid is used.

Effervescent base that can be used in the effervescent formulation according to the present invention can be selected from sodium hydrogen carbonate, sodium carbonate, sodium bicarbonate, potassium carbonate, potassium bicarbonate, potassium hydrogen carbonate, sodium glycine carbonate, lysine carbonate, arginine carbonate and calcium carbonate. Preferably, sodium hydrogen carbonate is used.

Sweetener and/or taste regulator that can be used in effervescent formulations of the present invention can be selected from, but not limited with, a group containing acesulfame, aspartame, dextrose, fructose, sucralose, sucrose, saccharin, saccharin sodium, lactitol, maltitol, maltose, sorbitol, sodium cyclamate, sucrose and xylitol or a combination thereof.

Flavoring agent that can be used in effervescent formulations of the present invention can be selected from, but not limited with, natural aroma oils (peppermint oil, oil of wintergreen, oil of cloves, parsley oil, eucalyptus oil, lemon oil, orange oil), menthol, menthane, anethole, methyl salicylate, eucalyptole, cinnamon, 1-methyl acetate, sage, eugenol, oxanon, alpha-irison, marjoram, lemon, orange, blackberry, propenyl guaethol acethyl, cinnamon, vanilla, thymol, linalol, cinnamalaldehyde glycerol acethal, N-quadric p-menthan-3-carboxamide, 3,1-methoxy propane 1,2-diol or a combination thereof.

Glidant that can be used in effervescent formulations of the present invention can be selected from, but not limited with, sodium lauryl sulfate, sodium benzoate, sodium chloride, sodium acetate, sodium acetate, sodium fumarate, carbowax 4000, L-leucine(17), PEG or a combination thereof.

Diluent that can be used in effervescent formulations of the present invention can be selected from, but not limited with, a group comprising calcium carbonate, calcium sulfate, dibasic calcium phosphate, tribasic calcium phosphate, calcium sulfate, microcrystalline cellulose, lactose, magnesium carbonate, magnesium oxide, maltodextrin, maltose, mannitol, sodium chloride, sorbitol, starch and xylitol or combinations thereof.

Disintegrant that can be used in effervescent formulations of the present invention can be selected from, but not limited with, a group comprising carboxymethyl cellulose calcium, carboxymethyl cellulose sodium, microcrystalline cellulose, silicone dioxide, croscarmellose sodium, crospovidone, hydroxypropyl cellulose, methyl cellulose, povidone, magnesium aluminium silicate and starch or a combination thereof.

Coloring agent that can be used in effervescent formulations of the present invention can be selected from, but not limited with, caratenoids or chlorophyl or a combination thereof.

Surfactant that can be used in effervescent formulations of the present invention can be selected from, but not limited with, sodium lauryl sulfate, sodium sulfate anhydrous and magnesium lauryl sulfate and a combination thereof.

Anti-foaming agent that can be used in effervescent formulations of the present invention can be selected from, but not limited with, simethicone and dimethyl siloxane, silicone oil or a combination thereof.

Humectant that can be used in effervescent formulations of the present invention can be selected from, but not limited with, anhydrous sodium sulphate, silica gel and potassium carbonate or combinations thereof.

Acidic agent that can be used in effervescent formulations of the present invention can be selected from, but not limited with, acetic acid, citric acid, lactic acid, malic acid, phosphoric acid, propionic acid, tartaric acid or combinations thereof.

Basic agent that can be used in effervescent formulations of the present invention can be selected from, but not limited with, potassium carbonate, potassium bicarbonate, potassium citrate, potassium hydroxide, sodium carbonate, sodium bicarbonate, or combinations thereof.

The term "Effervescent couple" refers to use of acidic agent and basic agent together.

The term "Effervescent formulations" comprises effervescent tablets, effervescent granules and effervescent powder. Effervescent formulations comprising cefaclor in accordance with the present invention is preferably compressed in the form of tablets.

In the effervescent pharmaceutical composition according to the present invention, with respect to the total weight of unit dosage; cefaclor is present in an amount of 1-55%, effervescent acid in an amount of 5-80 %, effervescent base in an amount of 5-30 %, surfactant in an amount of 1-25 %, binder in an amount of 0.3-7 %, lubricant in an amount of 2-10 %, sweetener and/or taste regulator in an amount of 0.5-6% and flavoring agent in an amount of 1-5 % can be used.

In the effervescent cefaclor formulation in accordance with the present invention, optionally a second active agent can be used. The second active agent can be selected from beta lactamase and cephalasporins, preferably clavulanic acid or its pharmaceutically acceptable derivatives are used.

Clavulanic acid used in the effervescent cefaclor formulation in accordance with the present invention, can be present in the form of its solvates, hydrates, enetiomers, racemates, organic salts, inorganic salts, polymorphs, crystal and amorphous forms or in free form and/or as a combination of these. Preferably, potassium clavulanate is used.

Clavulanic acid and derivatives thereof (for example potasium clavulanate) are very sensitive to moisture. Therefore, in the pharmaceutical composition according to the present invention, potassium clavulanate is preferably used together with a humidity absorbing agent in a ratio of 1:1.

One or more than one of the following substances can be used as a humidity absorbing agent; silica; colloid silica, for instance colloidal silica anhydrous, magnesium trisilicate, powder cellulose, magnesium oxide, calcium silicate, Syloid®, starch, microcrystalline cellulose and talc.

In the effervescent cefaclor formulation in accordance with the present invention, potasium clavulanate is preferably used with syloid or microcrystalline cellulose in a ratio of 1:1.

In the effervescent cefaclor formulation in accordance with the present invention, with respect to the total weight of the unit dose, 1-85%, preferably 5-75%, of clavulanic acid or pharmaceutically acceptable salts, hydrates, solvates or a combination thereof in an amount equivalent to that can be used.

Another aspect of the present invention is that the pharmaceutical composition prepared according to the invention is used for the treatment of the diseases relative to the upper respiratory tract infections such as pharangitis, tonsillitis, otitis media; lower respiratory tract infections such as acute pneumonia, acute and chronic broncihia and urinary tract infections such as acute cystitis and cystourethritis.

Another aspect of the present invention is the process for the preparation of the effervescent formulations comprising cefaclor in accordance with the present invention, which comprises the steps of;
1. Obtaining the granulation solution by mixing at least one binder, at least one lubricant and deionized water
2. Sieving the effervescent acid and base and granulating them with the granulation solution
3. Adding granules cefaclor, sweetener and flavoring agent after drying and sieving the granules obtained in the second step and mixing them to obtain the final mixture.
,In cases where effervescent formulations comprising cefaclor comprise a second active agent, for example potassium clavulanate, in the process used for the preparation of said formulation; potassium clavulanate: humidity absorbing agent (1:1) mixture is added along with cefaclor, sweetener and flavoring agent, in step 3 described above.
The obtained final mixture is optionally compressed in the form of tablets in the tablet pressing machine or filled into sachets.
Accordingly, another aspect the present invention is use of granulation solution comprising with respect to the total weight of the unit dose, more than 2%, preferably 2-8%, more preferably 2-5% of lubricant and more than 0.3%, preferably 0.35-7%, more preferably 0.4-4% of binder in the effervescent formulations comprising cefaclor.
In another aspect, the inventors have found that the possible bitter taste of cefaclor can be prevented and the drug in the effervescent form can have a pleasent taste by adding two different sweeteners after drying stage in the process for the preparation of the effervescent formulations comprising cefaclor in accordance with the present invention.
Though not limited with these examples, effervescent formulations according to present invention can be prepared according to the examples given below.

### Example 1:

Formulation and process for preparation of effervescent tablet A granulation solution comprising binder, lubricant and deionized water is prepared. Effervescent acid, base and surfactant are granulated with the granulation solution by adding them into fluid bed dryer. Cefaclor, sweetener and flavoring agent are added into the obtained mixture and this mixture is dried. Finally, the dried mixture is compressed into tablets.

| Component name | % amount in unit dose |
|---|---|
| Cefaclor | 23 % |
| Effervescent acid | 42% |
| Effervescent base | 11.5 % |
| Surfactant | 14.25 % |
| Binder | 1% |
| Sweetener | 3 % |
| Lubricant | 2.75 % |
| Flavoring Agent | 2.5 % |

### Example 2:

Formulation and process for preparation of effervescent tablet A granulation solution comprising binder, lubricant and deionized water is prepared. Effervescent acid, base and surfactant are granulated with the granulation solution by adding them into fluid bed dryer. Cefaclor, potassium clavulanate:humidity absorbing agent (1:1) mixture, sweetener and flavoring agent are added into the obtained mixture and this mixture is dried. Finally, the dried mixture is compressed into tablets.

| Component name | % amount in unit dose |
|---|---|
| Cefaclor | 13 % |
| potassium clavulanate:humidity absorbing agent (1:1) mixture | 21 % |
| Effervescent acid | 26% |
| Effervescent base | 17% |
| Surfactant | 16% |
| Binder | 1 % |
| Sweetener | 0.5 % |
| Lubricant | 2.75 % |
| Flavoring Agent | 2.5 % |

## Claims

1. A pharmaceutical composition formulated in effervescent form comprising 2-10 % of lubricant and 0.3-8% of binder and additionally cefaclor as active agent **characterized in that** lubricant and binder in said amounts are used together in the granulation solution used in the preparation of the formulation.

2. A pharmaceutical composition according to claim 1, wherein granulation solution comprises 2-8% lubricant and 0.3-7 % binder.

3. A pharmaceutical composition according to claims 1-2, wherein binder is selected from a group comprising; alginic acid, chitosan, carbomer, carboxymethyl cellulose sodium, dextrin, hydroxyethyl cellulose, hydroxymethyl cellulose, hydroxypropyl cellulose ethyl cellulose, gelatine, hypromellose, magnesium aluminium silicate, maltodextrin, polyethylene oxide and povidone or a combination thereof.

4. A pharmaceutical composition according to claim 3, wherein povidone is used as binder.

5. A pharmaceutical composition according to claims 1-4, wherein lubricant is selected from a group comprising calcium stearate, magnesium stearate, polyethylene glycol, PEG 6000, polyvinyl alcohol, potassium benzoate, sodium benzoate.

6. A pharmaceutical composition according to claim 5, wherein PEG 6000 is used as lubricant.

7. A pharmaceutical composition according to claims 1-6, wherein said composition is in the form of effervescent tablet.

8. A pharmaceutical composition according to claims 1-7, wherein cefaclor used as active agent is present in the form of its salts, hydrates, solvates, preferably monohydrate, dihydrate, trihydrate and/or anhydrous, even more preferably monohydrate form.

9. A pharmaceutical composition according to claims 1-8, wherein said composition comprises cefaclor, binder, lubricant and additionally at least one excipient which is selected from a group comprising effervescent couple containing effervescent acid and base, sweetener and/or taste regulator, flavoring agent, glidant, diluents, disintegrant, coloring agent, surfactant, anti-foaming agent, humectants, acidic agent, basic agent.

10. A pharmaceutical composition according to claim 9, wherein effervescent acid is selected from a group comprising citric acid, tartaric acid, malic acid, fumaric acid, ascorbic acid, acetic acid, adipic acid, succinic acid, acetylsalicylic acid and/or sodium citrate, sodium acetate, sodium dihydrogen phosphate, sodium acid pyrophosphate and sodium acid sulphite and
effervescent base is selected from sodium hydrogen carbonate, sodium carbonate, sodium bicarbonate, potassium carbonate, potassium bicarbonate, potassium hydrogen carbonate, sodium glycine carbonate, lysine carbonate, arginine carbonate and calcium carbonate.

11. A pharmaceutical formulation according to claim 10, wherein citric acid is used as effervescent acid and sodium hydrogen carbonate is used as effervescent base.

12. A pharmaceutical composition described in any of the previous claims, wherein cefaclor in an amount of 1-55%, effervescent acid in an amount of 5-80 %, effervescent base in an amount of 5-30 %, surfactant in an amount of 1-25 %, binder in an amount of 0.3-7 %, lubricant in an amount of 2-10 %, sweetener and/or taste regulator in an amount of 0.5-6% and flavoring agent in an amount of 1-5 % by total weight of unit dosage are used in said composition.

13. An effervescent formulation comprising cefaclor according to any of the preceding claims, wherein a second active agent which is selected from beta lactamase and/or cephalosporins preferably clavulanic acid and/or clavulanic acid derivative, even more preferably potassium clavulanate is used as a second active agent.

14. A process for the preparation of the pharmaceutical composition described in claims 1-13, wherein said process comprises the steps of obtaining the granulation solution by mixing at least one binder, at least one lubricant and deionized water; granulating the effervescent acid and base by the prepared granulation solution after sieving them; after drying and sieving the obtained granules, adding cefaclor, sweetener and flavoring agent to these granules.

15. A process according to claim 14, wherein two different sweeteners are added to the granules with cefaclor active agent after drying.

## Patentansprüche

1. Eine pharmazeutische Zusammensetzung formuliert in übersprudelnder Form bestehend aus 2-10 % von Schmierstoffe und 0.3-8 % von Bindemittel und zusätzlich Cefaclor als aktives Mittel **gekennzeichnet dadurch, daß** Schmierstoffe und Bindemittel in besagten Mengen sind verwendet zusammen in Granulationslösung verwendet in der Vorbereitung der Formulierung.

2. Eine pharmazeutische Zusammensetzung nach Anspruch 1, wo Granulationslösung beinhaltet 2-8 % Schmierstoff und 0.3-7 % Bindemittel.

3. Eine pharmazeutische Zusammensetzung nach Anspruch 1-2, wo Bindemittel ist ausgewählt von einer Gruppe bestehend aus Alginsäure, Chitosan, Carbomer, Carboxymethylzellulosenatrium, Dextrine, Hydroxyethylzellulose, Hydroxymethyl zellulose, Hydroxypropylzellulose, Ethylzellulose, Gelatine, Hypromellose, Magnesiumaluminiumsilikat, Maltodextrin, Polyethylenoxid und Povidon oder eine Kombination davon.

4. Eine pharmazeutische Zusammensetzung nach Anspruch 3, wo Povidon als Bindemittel verwendet wird.

5. Eine pharmazeutische Zusammensetzung nach Ansprüche 1-4, wo Schmiermittel ist ausgewählt von einer Gruppe bestehend aus Calciumstearat, Magnesiumstearat, Polyethyleneglycol, PEG 6000, Polyvinylalkohol, Kaliumbenzoate, Natriumbenzoate.

6. Eine pharmazeutische Zusammensetzung nach Anspruch 5, wo PEG 6000 verwendet wird als Schmierstoff.

7. Eine pharmazeutische Zusammensetzung nach Ansprüche 1-6, wo besagte Zusammensetzung ist in der Form von übersprudelnder Tablette.

8. Eine pharmazeutische Zusammensetzung nach Ansprüche 1-7, wo Cefaclor verwendet wird als aktives Mittel vorhanden in der Form von ihren Salze, Hydrate, Solvate, bevorzugt Monohydrate, Dihydrate, Trihydrate und/oder wasserfrei, sogar mehr bevorzugt Monohydrate Form.

9. Eine pharmazeutische Zusammensetzung nach Ansprüche 1-8, wo besagte Zusammensetzung beinhaltet Cefaclor, Bindemittel, Schmierstoffe und zusätzlich mindestens ein Arzneistoffträger, der ausgewählt ist von einer Gruppe bestehend aus übersprudelndes Paar beinhaltet übersprudelnde Säure und Base, Süßstoffe und/oder Geschmacksregler, Aromastoff, Glidant, Verdünnungsmittel, Sprengmittel, Farbstoff, Netzmittel, Antischaummittel, Feuchthaltemittel, saure Mittel, basische Mittel.

10. Eine pharmazeutische Zusammensetzung nach Ansprüche 9, wo übersprudelnde Säure ist ausgewählt von einer Gruppe bestehend aus Zitronensäure, Weinsäure, Apfelsäure, Fumarsäure, Ascorbinsäure, Essigsäure, Adipinsäure, Bernsteinsäure, Acetylsalicylsäure und/oder Natriumcitrat, Natriumacetat, Natriumdihydrogenphosphat, Natriumsäurepyrophosphat und Natriumsäuresulfit und
übersprudelnde Base ist ausgewählt von Natriumhydrogencarbonate, Natriumcarbonate, Natriumbicarbonate, Kaliumcarbonate, Kaliumbicarbonate, Kaliumhydrogencarbonate, Kaliumglycinecarbonate, Lysinecarbonate, Arginincarbonat und Calciumcarbonate.

11. **Eine** pharmazeutische Formulierung nach Anspruch 10, wo Zitronensäure verwendet wird als übersprudelnde Säure und Natriumhydrogencarbonate verwendet wird als übersprudelnde Base.

12. Eine pharmazeutische Zusammensetzung beschrieben nach den vorhergehenden Ansprüche, wo Cefaclor in einer Menge von 1-55%, übersprudelnde Säure in einer Menge von 5-80%, übersprudelnde Base in einer Menge von 5-30%, Netzmittel in einer Menge von 1-25%, Bindemittel in einer Menge von 0.3-7%, Schmierstoffe in einer Menge von 2-10%, Süßstoffe und/oder Geschmacksregler in einer Menge von 0.5-6% und Aromastoffe in einer Menge von 1-5% nach totalem Gewicht der Einheitsdose verwendet werden in besagter Zusammensetzung.

13. Eine übersprudelnde Formulierung bestehend aus Cefaclor nach den vorhergehenden Ansprüche, wo ein zweites aktives Mittel, das ausgewählt ist von beta lactamase und/oder Cephalosporin bevorzugt Clavulansäure und/oder Derivate der Clavulansäure, sogar mehr bevorzugt Kalium clavulanat wird verwendet als ein zweites aktives Mittel.

14. Ein Prozess für die Vorbereitung von pharmazeutischer Zusammensetzung beschrieben in Ansprüche 1-13, wo besagter Prozess beinhaltet Schritte der Erhaltung von Granulationslösung durch Einmischung von mindestens einem Bindemittel, mindestens einem Schmierstoff und deionisiertes Wasser; granuliert übersprudelnde Säure und Base durch vorbereitete Granulationslösung nach ihrer Siebung; nach Trocknen und Sieben der erhaltenen Körnchen, fügt hinzu Cefaclor, Süßstoffe und Aromastoffe zu diesen Körnchen.

15. Ein Prozess nach Anspruch 14, wo zwei verschiedene Süßstoffe hinzugefügt werden zu den Körnchen mit Cefaclor aktives Mittel nach Trocknen.

## Revendications

1. Composition pharmaceutique formulée sous forme effervescente comprenant 2-10% de lubrifiant et 0.3-8% de liant et de plus du céfaclor en tant qu'agent actif, **caractérisée en ce que** le lubrifiant et le liant dans ces quantités sont utilisés ensemble dans la solution de granulation utilisée dans la préparation de la formulation.

2. Composition pharmaceutique selon la revendication 1, dans laquelle la solution de granulation comprend 2-8% de lubrifiant et 0.3-7% de liant.

3. Composition pharmaceutique selon les revendications 1-2, dans laquelle le liant est choisi dans un groupe comprenant: l'acide alginique, le chitosane, le carbomère, la carboxyméthylcellulose, le sodium, la dextrine, l'hydroxyéthylcellulose, l'hydroxyméthylcellulose, l'hydroxypropylcellulose, l'éthylcellulose, la gélatine, l'hypromellose, le silicate de magnésium-aluminium, la maltodextrine, l'oxyde de polyéthylène et la povidone ou une combinaison de ceux-ci.

4. Composition pharmaceutique selon la revendication 3, dans laquelle la povidone est utilisée comme liant.

5. Composition pharmaceutique selon les revendications 1-4, dans laquelle le lubrifiant est choisi dans un groupe comprenant le stéarate de calcium, le stéarate de magnésium, le polyéthylèneglycol, le PEG 6000, l'alcool polyvinylique, le benzoate de potassium, le benzoate de sodium.

6. Composition pharmaceutique selon la revendication 5, dans laquelle le PEG 6000 est utilisé comme lubrifiant.

7. Composition pharmaceutique selon les revendications 1-6, dans laquelle ladite composition est sous la forme d'un comprimé effervescent.

8. Composition pharmaceutique selon les revendications 1-7, dans laquelle le céfaclor utilisé comme agent actif est présent sous la forme de ses sels, hydrates, solvates, de préférence monohydraté, dihydraté, trihydraté et/ou anhydre, encore plus préférentiellement sous forme monohydratée.

9. Composition pharmaceutique selon les revendications 1-8, dans laquelle ladite composition comprend du céfaclor, le liant, le lubrifiant et en outre au moins un excipient choisi dans un groupe comprenant un couple effervescent contenant un acide effervescent et une base, un édulcorant et/ou un régulateur de goût, un agent aromatisant, agent de glissement, diluants, désintégrant, colorant, tensioactif, agent antimousse, humectants, agent acide, agent basique.

10. Composition pharmaceutique selon la revendication 9, dans laquelle l'acide effervescent est choisi parmi l'acide citrique, l'acide tartrique, l'acide malique, l'acide fumarique, l'acide ascorbique, l'acide acétique, l'acide adipique, l'acide succinique, l'acide acétylsalicylique et / ou le citrate de sodium acétate, dihydrogénophosphate de sodium, pyrophosphate acide de sodium et sulfite acide de sodium et base effervescente est choisi parmi l'hydrogénocarbonate de sodium, le carbonate de sodium, le bicarbonate de sodium, le carbonate de potassium, le bicarbonate de potassium, l'hydrogénocarbonate de potassium, le carbonate de lysine, le carbonate d'arginine carbonate.

11. Composition pharmaceutique selon la revendication 10, dans laquelle l'acide citrique est utilisé comme acide effervescent et l'hydrogénocarbonate de sodium est utilisé comme base effervescente.

12. Composition pharmaceutique selon l'une quelconque des revendications précédentes, dans laquelle le céfaclore dans une quantité de 1-55%, l'acide effervescent dans une quantité de 5-80%, la base effervescente dans une quantité de 5-30%, le tensioactif dans une quantité de 1-25%, liant dans une proportion de 0.3-7%, lubrifiant dans une quantité de 2-10%, édulcorant et / ou régulateur de goût dans une quantité de 0.5-6% et agent aromatisant dans une quantité de 1-5% par le poids total de dosage unitaire est utilisé dans ladite composition.

13. Formulation effervescente comprenant du céfaclor selon l'une quelconque des revendications précédentes, dans laquelle un second agent actif choisi parmi la bêta lactamase et / ou les céphalosporines est de préférence l'acide clavulanique et / ou le dérivé d'acide clavulanique, encore plus préférablement le clavulanate de potassium.

14. Procédé pour la préparation de la composition pharmaceutique décrite dans les revendications 1-13, dans lequel ledit procédé comprend les étapes d'obtention de la solution de granulation en mélangeant au moins un liant, au moins un lubrifiant et de l'eau désionisée; granuler l'acide et la base effervescents par la solution de granulation préparée après les avoir tamisés; après séchage et tamisage des granules obtenus, ajout de céfaclor, édulcorant et agent aromatisant à ces granules.

15. Procédé selon la revendication 14, dans lequel deux édulcorants différents sont ajoutés aux granules avec un agent actif céfaclor après séchage.
